Europäisches Patentamt

(19) European Patent Office

Office européen des brevets

(11) Publication number : **0 404 750 B1**

# EUROPEAN PATENT SPECIFICATION

(45) Date of publication of patent specification :
**10.08.94 Bulletin 94/32**

(51) Int. Cl.[5] : **C07K 15/06**

(21) Application number : **90870075.0**

(22) Date of filing : **21.05.90**

(54) **Tissue inhibitor of metalloproteases (TIMP-2).**

(30) Priority : **26.05.89 US 358043**

(43) Date of publication of application :
**27.12.90 Bulletin 90/52**

(45) Publication of the grant of the patent :
**10.08.94 Bulletin 94/32**

(84) Designated Contracting States :
**AT BE CH DE DK ES FR GB GR IT LI LU NL SE**

(56) References cited :
THE JOURNAL OF BIOLOGICAL CHEMISTRY, vol. 261, no. 6, 25th February 1986, pages2814-2818, The American Society of Biological Chemists, Inc., Baltimore, US; G.S. HERRON et al.: "Secretion of metalloproteinases by stimulated capillaryendothelial cells"
THE JOURNAL OF BIOLOGICAL CHEMISTRY, vol. 261, no. 9, 25th March 1986, pages4154-4159, The American Society of Biological Chemists, Inc., Baltimore, US; J.B. MURRAY et al.: "Purification and partial amino acid sequence of a bovinecartilage-derived collagenase inhibitor"
BIOCHIMICA AND BIOPHYSICA ACTA, vol. 839, 1985, pages 214-218, Elsevier SciencePublishers B.V. (Biomedical Division), Amsterdam, NL; G. MURPHY et al.: "Tissueinhibitor of metalloproteinases. Identification of precursor forms synthesizedby human fibroblasts in culture"
EUROP. J. BIOCHEMISTRY, vol. 130, 1983, pages 79-83, FEBS, Berlin, DE; H.W.MACARTNEY et al.: "The collagenase inhibitor from human polymorphonuclearleukocytes isolation, purification and characterisation"
PROC. NATL. ACAD. SCI. USA, vol. 86, 1989, pages 8207-8211, Washington, US; G.I. GOLDBERG et al.: "Human 72-kilodalton type IV collagenase forms a complexwith a tissue inhibitor of metalloproteases designated TIMP-2"

(56) References cited :
JOURNAL OF BIOLOGICAL CHEMISTRY, vol. 264, no. 29, 15th October 1989, pages17374-17378, Baltimore, US; W.G. STETLER-STEVENSON et al.: "Tissue inhibitor ofmetalloproteinase (TIMP-2)"
THE JOURNAL OF BIOLOGICAL CHEMISTRY, vol. 264, no. 29, 15th October 1989, pages17445-17453, The American Society of Biochemistry and Molecular Biology, Inc.,Baltimore, US; Y.A. DE CLERCK et al.: "Purification and characterization of two related but distinct metalloproteinase inhibitors secreted by bovine aorticendothelial cells"

(73) Proprietor : **WASHINGTON UNIVERSITY**
Campus Box 1137,
1 Brookings Drive
St. Louis, Missouri 63130-4899 (US)

(72) Inventor : **Goldberg, Gregory Isaac**
4111 West Pine, No. 10
St Louis, Missouri 63108 (US)

(74) Representative : **Ernst, Hubert G. et al**
Monsanto Services International S.A.
Letter Box 21
Avenue de Tervuren 270-272
B-1150 Brussels (BE)

EP 0 404 750 B1

## Description

### Background of the Invention

This invention relates to a tissue inhibitor of metalloproteases. More particularly, this invention relates to a novel tissue inhibitor of metalloproteases, referred to herein as TIMP-2, which forms an exclusive complex with the 72-kDa type IV collagenase.

Tissue inhibitor of metalloproteases (TIMP) is a ubiquitous glycoprotein with an apparent molecular weight of approximately 30,000 which was originally purified from serum-free medium conditioned by normal human skin fibroblasts. See Welgus and Stricklin, J. Biol. Chem. 258, 12259-12264 (1983); Welgus et al., Ibid. 254, 1938-1943 (1979). Inhibition of interstitial collagenase is stoichiometric, with a 1:1 molar ratio of inhibitor to enzyme being required for complete inhibition of enzyme activity. Interstitial procollagenase can neither interact with TIMP nor bind to collagen. Substrate binding and interaction with inhibitor both require extracellular activation of the collagenase zymogen. Active enzyme binds with nearly equal affinity to both monomeric collagen and aggregated fibrils. The $K_i$ of enzyme-inhibitor binding was determined to be less than $10^{-9}$M. See Welgus et al., Collagen Rel. Res. 5, 167-179 (1985).

TIMP was cloned simultaneously as a collagenase inhibitor [Carmichael et al., Proc. Natl. Acad. Sci. 83, 2407-2411 (1986); Andrew et al., Nature 318, 66-69 (1985)] and a factor with erythroid potentiating activity (EPA) [Gasson et al., Ibid. 315, 768-771 (1985)] which stimulates colony formation by relatively mature erythroid precursors (CFU-E). The TIMP gene was localized to the X chromosome. See Mullins et al., Genomics 3, 187-194 (1988); Mahtani and Willard, Ibid. 2 294-301 (1988). The secreted protein consists of 184 amino acid residues and contains six disulfide bonds and two glycosylation sites containing N-linked oligosaccharides. The sequence of the inhibitor has no substantial homology to previously sequenced protease inhibitors with the exception of the metalloprotease inhibitor isolated from bovine scapular cartilage [Murray et al., J. Biol. Chem. 261, 4154-4159 (1986)] and from normal murine fibroblasts [Edwards et al., Nucl. Acids Res. 14, 8863-8878 (1986); Coulombe and Skup, J. Biol. Chem. 263, 1439-1443 (1988)]. The inhibitor extracted from cartilage has a molecular mass of 27,400 with an $NH_2$-terminal sequence of 45 residues that shows 65% homology to TIMP.

Recent observations suggest that expression of TIMP may be involved in suppression of the tumorigenicity of immortal murine 3T3 cells. See Khokha et al., Science 243, 947-950 (1989). Mouse 3T3 cell lines constitutively synthesizing an RNA complementary to the messenger RNA encoding TIMP were shown to secrete a reduced amount of TIMP into the culture medium. Unlike the parental cells, these cells were invasive in a human amnion invasion assay and were tumorigenic and metastatic in athymic mice. In addition, in an in vitro amnion invasion assay system, TIMP inhibited the invasion of B16-F10 murine melanoma cells through the human amniotic membrane [Schultz et al., Cancer Res. 48, 5539-5545 (1988)] while intraperitoneal injection of recombinant TIMP (rTIMP) into mice showed a significant inhibition of metastatic lung colonization by these cells. The anticolonization effect of rTIMP was found to be due to its action on invasion rather than on tumor growth.

### Brief Description of the Invention

In accordance with the present invention, a novel tissue inhibitor of metalloproteases is provided. This novel inhibitor also is referred to herein for convenience as TIMP-2. Although TIMP-2 is structurally related to the previously described TIMP, it unexpectedly forms an exclusive complex with the 72-kDa type IV collagenase whereas TIMP complexes exclusively with the 92-kDa type IV collagenase.

TIMP-2 is a 24-kDa inhibitor. It is homologous to TIMP as demonstrated by comparison of the partial amino acid sequence of this protein to TIMP, although it does not cross-react with TIMP specific antibody.

The 72-kDa type IV collagenase - TIMP-2 complex can be activated with organomercurials to yield a catalytically competent enzyme. Activation occurs concomitantly with autoproteolytic cleavage of the amino terminus of the protein and does not require dissociation of the complex. Surprisingly, both activity and activation of the complex can be completely inhibited by further addition of stoichiometric quantities of purified TIMP-2 or recombinant TIMP, suggesting that the new inhibitor can bind to the 72-kDa type IV collagenase in a 2:1 molar ratio rendering the enzyme inactive.

The 72-kDa type IV procollagenase purified from each of H-ras transformed human bronchial epthelial cells, SV-40 transformed human lung fibroblasts and normal skin fibroblasts exists in a stable but non-covalent stoichiometric complex with the novel TIMP-2 inhibitor of this invention.

The 92-kDa type-IV collagenase and its complexing with TIMP are disclosed in a copending application assigned to a common assignee. The 72-kDa type IV collagenase is disclosed by Collier et al., J. Biol. Chem. 263, 6579-6587 (1988), and in another copending application.

### Detailed Description of the invention

While the specification concludes with claims particularly pointing out and distinctly claiming the subject matter regarded as forming the present invention, it is believed that the invention will be better understood from the following detailed description of preferred embodiments taken in conjunction with the appended drawings, in which;

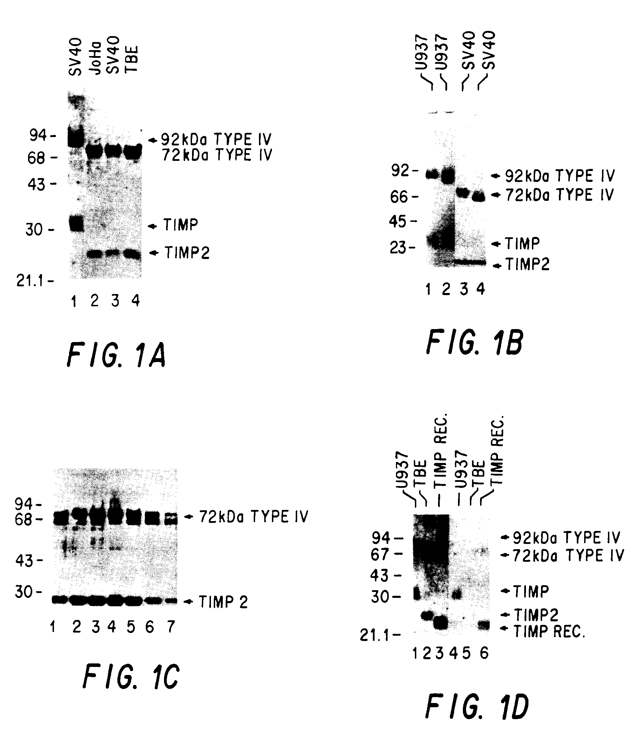

FIG. 1 shows a series of electrophoretic gel patterns which provide an analysis of the type IV collagenase - inhibitor complex.

A. One µg samples of purified type IV collagenase - inhibitor complex from the indicated cell sources (SV-40, JoHa, TBE) were subjected to Na-DodSO$_4$/PAGE on a 13% gel.

B. The enzyme - inhibitor complex as in A before (lanes 1,3) and after (lanes 2,4), activation with p-aminophenylmercuric acetate (APMA).

C. An APMA activated 72-kDa type IV collagenase complex was size fractionated on an AcA-44 gel filtration column and fractions were analyzed on silver stained 13% NaDodSO$_4$/PAGE.

D. Purified complex (lanes 1, 2, 4, 5) and purified recombinant (REC) TIMP (lanes 3,6) were stained (lanes 1-3), or subjected to western blot analysis using anti-TIMP antibody.

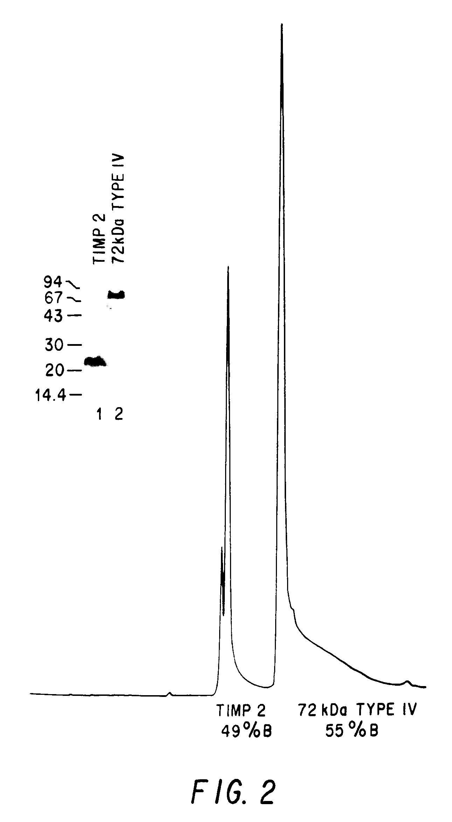

FIG. 2 shows the reverse phase HPLC of the 72-kDa type IV procollagenase - TIMP-2 complex. A purified preparation of the 72-kDa type IV collagenase - TIMP-2 complex from TBE cells was chromatographed on 130A Microbore Separation System as described hereinafter under "Materials and Methods." Each peak was collected, lyophilized and analyzed on silver stained NaDodSO$_4$/PAGE (see insert).

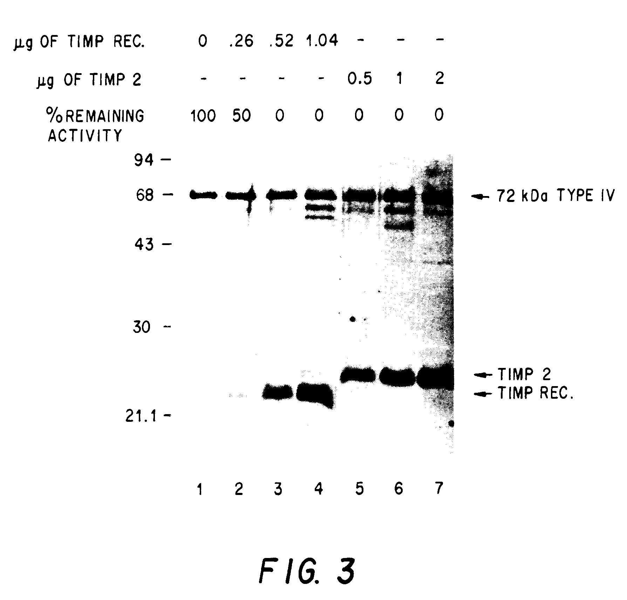

FIG. 3 shows the activation of 72-kDa type IV procollagenase - TIMP-2 complex and inhibition of its activity with TIMP and TIMP-2. The enzyme-inhibitor complex purified from TBE cells was activated with 1 mM APMA as described hereinafter under "Materials and Methods" (lane 1), briefly incubated with the indicated amounts of recombinant TIMP (lanes 2-4) or purified TIMP-2 (lanes 5-7). The reaction mix was separated into two aliquots and electrophoresed on a 13% NaDodSO$_4$ gel or assayed for gelatinolytic activity against $^{14}$C- gelatin (see "Materials and Methods").

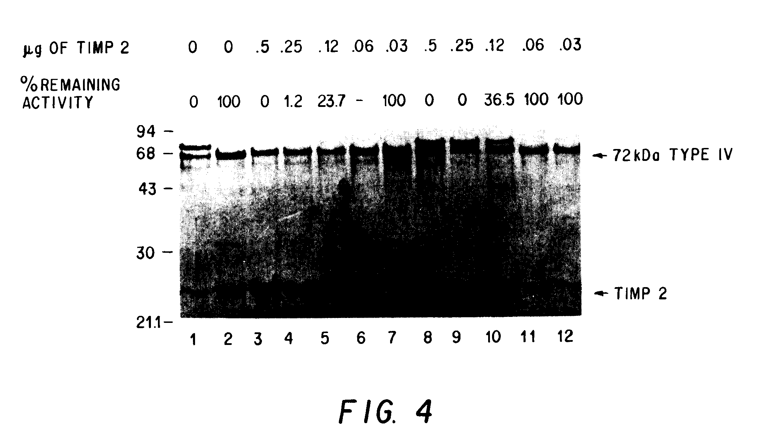

FIG. 4 shows the activation of 72-kDa type IV procollagenase - TIMP-2 complex and inhibition of its activity by the further addition of TIMP-2. 0.5 µg of the proenzyme - inhibitor complex purified from TBE cells (lane 1) was activated with 1 mM APMA (lane 2), with (lanes 8-12), or without (lanes 3-7) indicated additional amounts of TIMP-2. Samples in lanes 3-7 were briefly incubated with indicated amounts of purified TIMP-2 after activation. Samples were analyzed as in FIG. 3.

Amino acids are shown herein either by three letter or one letter abbreviations as follows:

| Abbreviated Designation | | Amino Acid |
| --- | --- | --- |
| A | Ala | Alanine |
| C | Cys | Cysteine |
| D | Asp | Aspartic acid |
| E | Glu | Glutamic acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophan |
| Y | Tyr | Tyrosine |

In order to illustrate specific preferred embodiments of the invention in greater detail, the following exemplary laboratory preparative work was carried out.

EXAMPLE 1

MATERIALS AND METHODS

Cell Culture.

SV-40 transformed fetal lung fibroblasts and the parental line IMR-90 (from the NIH Institute for Aging, Bethesda) were cultured in Eagle's minimum essential medium containing 29 µg/ml of glutamine and 10% fetal calf serum. The monocytic leukemia strain U-937 (from the Washington University School of Medicine Cancer Center) was cultured in RPMI-1640 medium containing 5% fetal calf serum. U-937 cells ($2 \times 10^6$ cells/ml) were treated with 12-O-tetradecanoylphorbol acetate (TPA) (50 ng/ml) in Dulbecco's modified Eagle's/F-12 medium containing 0.1% fetal serum for 24-48 hours. The cell lines IMR-90 and U-937 also are both available from the American Type Culture Collection, Rockville, Maryland, in unrestricted deposits under accession numbers ATCC CCL 186 and ATCC CRL 1593, respectively.

Enzyme Purification.

The 92-kDa and 72-kDa type IV collagenases were purified from conditioned medium of SV-40 trans-

4

formed fibroblasts or TPA-differentiated U-937 cells using a three-step procedure. Serum-free medium was adjusted to 0.01 M Tris-HCl, pH 7.5, and applied to a 2.5 x 10 cm column of reactive red Agarose (Sigma) equilibrated in 0.02 M Tris-HCl pH 7.5, containing 0.005 M $CaCl_2$ (Tris/$CaCl_2$ Buffer) and 0.15 M NaCl. The enzyme was eluted from the column using a 0.15 to 2.0 M NaCl linear gradient in Tris/$CaCl_2$ Buffer and fractions were assayed by gelatin zymography [Heussen and Dowdle, Anal. Biochem. 102, 196-202 (1980)].

Fractions containing gelatinolytic activity were pooled, adjusted to 0.5 M $NaCl_2$ and 0.01% Brij®-35 detergent and chromatographed on a 1.0 x 10 cm column of gelatin-Sepharose® (Sigma) as previously described [Collier et al., J. Biol. Chem. 263, 6579-6581 (1988); Hibbs et al., Ibid. 260, 2493-2500 (1985)] except the enzyme was eluted using a 0-10% dimethylsulfoxide (DMSO) gradient which permitted resolution of two enzymes. Gelatin-Sepharose purified enzyme was dialyzed into 0.005 M Tris-HCl, pH 7.5, buffer containing 0.0001 M $CaCl_2$ and 0.005% Brij-35 and stored at -80°C. An optional step includes gel-filtration chromatography on an AcA-44 (IBS Biotechnics) column equilibrated in Tris/$CaCl_2$ buffer containing 1.0 M NaCl and 0.01% Brij-35.

Purification of TIMP-2 and Sequence Analysis.

The purified 72-kDa type IV collagenase - TIMP-2 complex was subjected to gel-filtration in 0.02 M Tris-HCl, pH 7.5, 0.15 M NaCl buffer containing 0.1% $NaDodSO_4$ in order to dissociate the proenzyme - TIMP complex. The fractions containing TIMP-2 were pooled and dialyzed to equilibrium against 0.005 M Tris-HCl, pH 7.5, containing 0.0001 M $CaCl_2$, and 0.005% Brij-35 (LB buffer).

The gelatin-Sepharose purified enzyme-inhibitor complex was subjected to reverse-phase HPLC using an Applied Biosystems 130A Microbore separation system with a 2.1 x 30 mm column equilibrated in 0.07% trifluoroacetic acid (TFA). The column was developed with a linear gradient of 0-70% acetonitrile in 0.07% TFA. HPLC purified TIMP-2 was identified by silver staining of the Na-DodSO₄/PAGE, lyophilized and subjected to S-pyridylethylation (Applied Biosystems User Bulletin, #28, 470A/477A-120A). The protein was reconstituted in 6 M guanidine-HCl, 0.25 M Tris-HCl, pH 8.5, containing 0.5%-2 mercaptoethanol and incubated for 2 h in the dark under nitrogen. One part 4-vinylpyridine was then added to 25 parts of the protein solution. The mix was incubated as above and then desalted on the same HPLC system. The TIMP-2 peak was collected, reconstituted in 50 mM ammonium bicarbonate and digested with TPCK-trypsin (Worthington). The peptides were separated by Microbore reverse-phase HPLC as described above. Several of the resolved peptides and the untreated TIMP-2 were subjected to amino acid sequence determination using an Applied Biosystems 470A gas phase sequencer.

Enzyme Assays.

Samples of purified proenzyme in LB buffer were adjusted to 0.25 M Tris-HCl, pH 7.5, 0.025 M NaCl, 0.01% Brij-35 and activated by incubating the proenzyme for 1 h at 37°C with 0.001 M of the organomercurial compound, p-aminophenylmercuric acetate (APMA) dissolved in 0.05 N NaOH. The activated enzyme was preincubated with inhibitor as indicated for 5 min at 25°C and the samples divided into equal aliquots. Enzyme activity, using $^{14}$C-gelatin (40,000 cpm/mg) as the substrate, was determined as described previously by Collier et al., Supra. and Wilhelm et al., Proc. Natl. Acad. Sci. USA 84, 6725-6729 (1987). The other aliquot was subjected to Na-DodSO₄/PAGE analysis and laser densitometry. Alternatively, inhibitor was added prior to the addition of APMA and activation was then initiated as described above.

RESULTS

A novel 24-kDa inhibitor, TIMP-2, was shown above to preferentially interact with 72-kDa type IV collagenase to form a stable stoichiometric complex.

Human bronchial epithelial cells secrete a 72-kDa type IV collagenase in response to transformation with H-ras (Collier et al., Supra). This metalloprotease is also secreted in large quantities by other human transformed cell lines as well as normal human skin fibroblasts. The 92-kDa enzyme is secreted by several transformed cell lines of fibroblast origin, and other tumor cells. This enzyme is normally expressed by human macrophages and epidermal keratinocytes in preference to the 72-kDa enzyme. In addition, SV-40 transformed human lung fibroblasts secrete both the 72-kDa type IV collagenase and the 92-kDa type IV collagenase which is not detectable in the parental cell line, IMR-90.

It has been demonstrated in a copending application that the 92-kDa type IV procollagenase exists in a non-covalent complex with TIMP, which can be activated by APMA, yielding an enzyme with a substrate specificity similar to that of the 72-kDa type IV collagenase. The complex has been purified from phorbol ester differentiated U-937 monocytic leukemia cells, the human fibrosarcoma cell strain, HT-1080, and SV-40 transformed human lung fibroblasts. The results presented in Fig. 1A demonstrate that the 92-kDa type IV collagenase purified from conditioned media of SV-40 cells contains stoichiometric amounts of the 30-kDa TIMP, whereas the 72-kDa type IV collagenase, purified from the same starting material contains a 24-kDa protein. This latter protein was not detected in any preparations of affinity purified 92-kDa enzyme. Conversely, TIMP was not detected in preparations of the 72-kDa enzyme, purified using the same procedure (see under "Materials and Methods"). This observation demonstrates that although the same cells secrete all four components involved in the formation of the two enzyme-inhibitor complexes, TIMP associates exclusively with the 92-kDa type IV procollagenase whereas the 24-kDa protein is found complexed with the 72-kDa enzyme.

The 24-kDa protein found in preparations of the type IV collagenase did not react with TIMP specific antibody (Fig. 1D), but both recombinant TIMP and the TIMP associated with the 92-kDa enzyme did react with anti-TIMP antibody. Gel filtration chromatography of the gelatin-Sepharose purified 72-kDa enzyme in 1.0 M NaCl (data not shown) failed to resolve the putative complex between these two proteins. However, gel-filtration in the presence of 0.1% Na-DodSO$_4$ did separate the proenzyme from the complexed protein. These observations indicate that the 72-kDa purified proenzyme and the 24-kDa protein exist in a stable non-covalent complex.

The results presented in Fig. 2 demonstrate the dissociation of the complex after acidification of the sample with TFA and HPLC chromatography. This procedure permits purification of the 24-kDa protein free of the enzyme as shown by the NaDodSO$_4$/PAGE analysis of the protein peaks eluted from the HPLC column (Fig. 2).

To obtain a partial amino acid sequence of the 24-kDa protein the first peak (Fig. 2) was collected, lyophilized and alkylated (see under "Materials and Methods"). The alkylated material was desalted on the same HPLC column, lyophilized and subjected to digestion with trypsin. The tryptic digest was further chromatographed as in Fig. 2. The resulting peptides were subjected to amino acid sequence analysis. Undigested protein was used to determine the sequence of the amino terminus. The determined peptide sequences are presented in Table 1, below. The homology between the sequences of these peptides and TIMP clearly demonstrates a close structural relationship of the two proteins. Based on this observation, the 24-kDa protein found in complex with the 72-kDa type IV collagenase is referred to as TIMP-2.

## Table 1

### Amino Acid Sequence of Peptides Derived from
### Human Inhibitor TIMP-2*

```
TIMP-2 NT :   SPVHPQQAFCNADVVIRAKAVS
TIMP       :   V P   T    S L    F    G

TIMP-2 TP1:   ITLCDFIVPWDTLSTTQK
TIMP       :     T S  VA   NS   LA R

TIMP-2 TP2:   CPMIPCYISSPDECL
TIMP       :     LS     KLQ GTH
```

* TIMP-2 was purified as in Fig. 2, subjected to S-pyridylethylation and digested with trypsin. Tryptic peptides (TP I and 2) were fractionated and sequenced as described under "Materials and Methods." Purified 72-kDa type IV collagenase - TIMP-2 complex was electroblotted onto a PVDF membrane and a protein band corresponding to TIMP-2 was subjected to $NH_2$-terminal sequence analysis (NT). Top line, amino acid sequence of the TIMP-2. Second line, human TIMP, showing amino acids which differ from TIMP-2.

APMA induced activation and enzymatic activity of the TIMP-2-type IV collagenase complex can be inhibited by further addition of stoichiometric amounts of TIMP-2.

Treatment of the 72-kDa type IV collagenase with organomercurials results in the proteolytic processing of the amino terminal domain with a loss of 6 kDa in molecular mass and conversion of the proenzyme into a catalytically competent form [Collier et al., J. Biol. Chem. 263, 6579-6587 (1988); Stetler-Stevenson et al., Ibid. 264, 1353-1356 (1989)]. The formation of a complex between TIMP-2 and the 72-kDa proenzyme did not prevent its activation by treatment with the organomercurial APMA (Fig. 1B). Similarly, autoactivation of TIMP - 92-kDa type IV collagenase complex, initiated by APMA, resulted in a loss of 8-kDa as determined by Na-DodSO$_4$/PAGE (Fig. 1B), corresponding to the cleavage of 73 amino acid residues from the amino terminal domain. Activation did not affect the dissociation of the enzyme-inhibitor complex, since gel-filtration chromatography of the activated enzyme failed to resolve the enzyme from TIMP-2 (Fig. 1C). The specific activity of the APMA-activated enzyme-TIMP-2 complex against $^{14}$C-gelatin was between 900 and 1200 units/mg of enzyme protein.

To investigate the possibility that TIMP-2 may inhibit 72-kDa type IV collagenase activity, a purified preparation of TIMP-2 was obtained and tested as follows. Purified 72-kDa type IV collagenase - TIMP-2 complex was dissociated and subjected to gel filtration chromatography on ACA-44 in presence of 0.1% Na-DodSO$_4$. The fractions containing free TIMP-2 were pooled after analysis on silver stained Na-DodSO$_4$/PAGE. The protein was then reconstituted using equilibrium dialysis against LM buffer containing 0.005% Triton® X-100 detergent. This preparation was used to study the effect of addition of TIMP-2 on the type IV collagenase-TIMP complex as shown in Figs. 3 and 4.

The enzyme was activated by treatment with 1 mM APMA for 1 h at 37°C with (Fig. 4 lanes 8-12) or without (Fig. 3 and Fig. 4 lanes 2-7) addition of the inhibitor in the amounts indicated. At the end of the activation reaction, inhibitor was added to the samples, which were activated in the absence of the inhibitor (Fig. 3, Fig. 4, lanes 2-7). Each sample was divided into two aliquots and each aliquot was assayed for activity using the $^{14}$C-gelatin as a substrate (see under "Materials and Methods"), or analyzed on Na-DodSO$_4$/PAGE. The gels were then scanned with a laser densitometer to quantitate the amount of TIMP-2 relative to the amount of enzyme present in the reaction. This permitted assessment of the extent of enzyme activation (by a shift in the apparent molecular weight) independent of the activity assay. The addition of a molar excess of TIMP-2 to the activated type IV collagenase - TIMP-2 complex completely inhibited its gelatinolytic activity (Figs. 3 and 4). Furthermore, presence of the same amounts of TIMP-2 during the APMA treatment inhibited activation since no conversion of the enzyme into a lower molecular mass form was observed, compared to control (Fig. 4, lane 1). In agreement with this result the gelatinolytic activity of the complex treated with APMA in the presence of excess of TIMP-2 was completely inhibited.

Inhibition of both APMA induced activation and gelatinolytic activity of the 72-kDa type IV collagenase - TIMP-2 complex required addition of stoichiometric quantities of free TIMP-2, rather than small amounts as compared to the amount of enzyme complex in the reaction mix. Although the 72-kDa type IV procollagenase was found complexed only to TIMP-2, the addition of recombinant TIMP to the complex resulted in complete inhibition of activity (Fig. 3, lanes 2-4). This result suggests the possibility that binding of two moles of TIMP-2 or one mole each of TIMP and TIMP-2 per one mole of the enzyme is required to achieve complete inhibition of activation and catalytic activity. These results are also consistent with the autoproteolytic mechanism of metalloprotease activation. See Grant et al., J. Biol. Chem. 262, 5886-5889 (1987).

## Claims

1. A protein which is a tissue inhibitor of metalloproteases and characterized as follows:
   (a) it has a molecular weight determined by NaDodSO$_4$/PAGE electrophoresis of about 24 kilodaltons,
   (b) it forms a stable non-covalent complex with the 72-kilodalton type IV collagenase but not with the 92-kilodalton type IV collagenase,
   (c) it is activated with organomercurials to form a catalytically competent enzyme,
   (d) it has a N-terminal amino acid sequence as follows:

   SPVHPQQAFCNADVVIRAKAVS,

   and
   (e) it has two internal amino acid partial sequences as determined by tryptic digestion of the full protein as follows:

   ITLCDFIVPWDTLSTTQK

   and

   CPMIPCYISSPDECL.

2. A method of inhibiting human type IV collagenase activity comprising subjecting a biological fluid containing said activity to an inhibitory effective amount of the protein of Claim 1.

## Patentansprüche

1. Protein, das ein Gewebeinhibitor für Metallproteasen ist und wie folgt gekennzeichnet ist:
   (a) es hat ein mittels NaDodSO$_4$/PAG-Elektrophorese bestimmtes Molekulargewicht von etwa 24 Kilodalton,
   (b) es bildet einen stabilen, nicht-kovalenten Komplex mit der 72-Kilodalton-Typ-IV-Collagenase, jedoch nicht mit der 92-Kilodalton-Typ-IV-Collagenase,
   (c) es wird durch Organoquecksilber-Verbindungen zur Bildung eines katalytisch kompetenten Enzyms aktiviert,
   (d) es hat eine N-terminale Aminosäuresequenz wie folgt:

   SPVHPQQAFCNADVVIRAKAVS,

   und

(e) es hat folgende zwei innere Aminosäure-Teilsequenzen, wie durch tryptischen Verdau des gesamten Proteins bestimmt:

$$ITLCDFIVPWDTLSTTQK$$

und

$$CPMIPCYISSPDECL.$$

2. Verfahren zur Inhibierung der Typ-IV-Humancollagenase-Aktivität, bei welchem eine biologische Flüssigkeit, welche diese Aktivität enthält, einer inhibierend wirksamen Menge des Proteins nach Anspruch 1 ausgesetzt wird.

## Revendications

1. Protéine qui est un inhibiteur tissulaire des métalloprotéases, et qui présente les caractéristiques suivantes :

a) sa masse moléculaire, déterminée par électrophorèse NaDodSO$_4$/PAGE, vaut environ 24 kilodaltons ;

b) elle forme un complexe stable non-covalent avec la collagénase de type IV de 72 kilodaltons, mais non pas avec la collagénase de type IV de 92 kilodaltons ;

c) elle est activée par les composés organomercuriels pour former une enzyme catalytiquement compétente ;

d) elle présente la séquence N-terminale suivante d'acides aminés :

$$SPVHPQQAFCNADVVIRAKAVS \quad ;$$

et

e) elle comporte les deux séquences partielles internes suivantes d'acides aminés, déterminées par digestion trypsique de la protéine entière :

$$ITLCDFIVPWDTLSTTQK$$

et

$$CPMIPCYISSPDECL.$$

2. Procédé d'inhibition de l'activité de la collagénase humaine de type IV, comportant le fait de soumettre un fluide biologique présentant cette activité à l'action d'une quantité efficacement inhibitrice d'une protéine conforme à la revendication 1.

**FIG. 1A**

**FIG. 1B**

**FIG. 1C**

**FIG. 1D**

FIG. 2

| μg OF TIMP REC. | 0 | .26 | .52 | 1.04 | - | - | - |
|---|---|---|---|---|---|---|---|
| μg OF TIMP 2 | - | - | - | - | 0.5 | 1 | 2 |
| %REMAINING ACTIVITY | 100 | 50 | 0 | 0 | 0 | 0 | 0 |

94 —

68 —  ← 72 kDa TYPE IV

43 —

30 —

← TIMP 2
← TIMP REC.

21.1 —

1  2  3  4  5  6  7

## FIG. 3

EP 0 404 750 B1

FIG. 4